# NEUE EUROPÄISCHE PATENTSCHRIFT

(11) **EP 1 098 696 B2**
(45) Veröffentlichungstag und Bekanntmachung des Hinweises auf die Entscheidung über den Einspruch: **14.07.2010**
(45) Hinweis auf die Patenterteilung: 07.11.2007
(21) Anmeldenummer: 99938268.2
(22) Anmeldetag: 15.07.1999
(51) Int. Cl.: B01J 13/02, B01J 13/22

(54) **POLYELEKTROLYTHÜLLEN AUF BIOLOGISCHEN TEMPLATEN**
POLYELECTROLYTE COVERINGS ON BIOLOGICAL TEMPLATES
REVETEMENT POLYELECTROLYTIQUE DE SPECIMENS BIOLOGIQUES

(30) Priorität: 15.07.1998 EP 98113181; 22.02.1999 DE 19907552
(43) Veröffentlichungstag der Anmeldung: 16.05.2001
(73) Patentinhaber: Max-Planck-Gesellschaft zur Förderung der Wissenschaften e.V., 80539 München (DE)
(72) Erfinder: NEU, Björn, D-10115 Berlin (DE); BÄUMLER, Hans, D-13187 Berlin (DE); DONATH, Edwin, D-16845 Giesenhorst (DE); MOYA, Sergio, D-14052 Berlin (DE); SUKHORUKOV, Gleb, Moscow District, 142292 (RU); MÖHWALD, Helmuth, D-55411 Bingen (DE); CARUSO, Frank, D-12161 Berlin (DE)
(74) Vertreter: Vossius & Partner
(86) Internationale Anmeldenummer: PCT/EP1999/005063
(87) Internationale Veröffentlichungsnummer: WO 2000/003797

(56) Entgegenhaltungen:
- EP-B1- 0 782 853
- WO-A-95/26714
- WO-A-96/18498
- WO-A-96/30409
- WO-A-98/14180
- WO-A1-93//16687
- DE-A- 4 026 978
- DE-A- 19 812 083
- DE-A1- 4 312 970
- CARUSO F.; DONATH E.; MÖHWALD H.: 'INFLUENCE OF POLYELECTROLYTE MULTILAYER COATINGS ON FOERSTER RESONANCE ENERGY TRANSFER BETWEEN 6-CARBOXYFLUORESCEIN AND RHODAMINE B-LABELED PARTICLES IN AQUEOUS SOLUTION MATERIALS' JOURNAL OF PHYSICAL CHEMISTRY B Bd. 102, 1998, WASHINGTON, Seiten 2011 - 2016, XP000852731
- ELSEVIER SCIENCE B.V.: 'Colloids and surfaces A:Physicochemical and Engineering Aspects 137', 1998, ELSEVIER SCIENCE B.V. Artikel G.B. SUKHORUKOV ET AL.: 'Layer-by-layer self assembly of polyelectrolytes on colloidal particles', XP000852906

## Beschreibung

Die Erfindung betrifft Verfahren zur Herstellung von Kapseln mit einer Polyelektrolythülle sowie die durch das Verfahren erhältlichen Kapseln.

Mikrokapseln sind in verschiedenen Ausführungsformen bekannt und werden insbesondere für die kontrollierte Freisetzung und den zielgerichteten Transport von pharmazeutischen Wirkstoffen sowie zum Schutz von empfindlichen Wirkstoffen, wie etwa Enzymen und Proteinen verwendet.

Mikrokapseln können durch mechanisch-physikalische Verfahren, bzw. Versprühen und nachfolgende Beschichtung, chemische Verfahren, wie etwa Grenzflächenpolymerisation bzw. -kondensation oder Polyrrierphasentrennung oder durch Verkapselung von Wirkstoffen in Liposomen hergestellt werden. Bisher bekannte Verfahren weisen jedoch eine Reihe von Nachteilen auf.

WO 95/26714 betrifft das Einkapseln lebender Zellen in einer Membran, bei der es sich um einen Komplex handelt, der durch Kohäsion von zwei Polymerschichten entsteht. Die innere Schicht umfasst ein Substratbiopolymer und die äußere Schicht einen synthetischen Polyelektrolyten mit einer zum Biopolymer entgegengesetzten Ladung. Bei dieser Vorgehensweise werden Polyelektrolytkapseln nicht auf den Zellen, sondern Tropfen mit darin suspendierten Zellen gebildet. Nachteilig bei diesem Verfahren ist, dass die Dicke der Membranen und die Größe der Mikrokapseln nur schwer kontrolliert werden kann.

WO 99/47252 A2 betrifft ein Verfahren zur Herstellung von Kapseln unter Verwendung von Templatpartikeln, wie z.B. teilvernetzten Melaminformaldehydpartikeln und Herstellen einer Hülle um die Partikel durch Aufbringen von Polyelektrolyten.

WO 99/47253 und EP 0 972 563 betreffen die Beschichtung von Templatpartikeln mit alternierenden Schichten aus entgegengesetzt geladenen Nanopartikeln und Polyelektrolyten bzw. alternierenden Schichten von entgegengesetzt geladenen Nanopartikeln.

F. Caruso et al., J. Phys. Chem. B 1998, 102, 2011-2016, beschreiben die Beschichtung von Rhodamin B-markierten nicht desintegrierbaren Melaminformaldehydpartikeln mit Polyelektrolytschichten. Die Multischichten werden durch FRET untersucht.

Die Deutsche Patentanmeldung 198 12 083.4 beschreibt ein Verfahren zur Herstellung von Mikrokapseln mit einem Durchmesser < 10 µm, wobei auf einer wässrigen Dispersion von Templatpartikeln mehrere aufeinanderfolgende Schichten entgegengesetzt geladener Poylelektrolytmoleküle aufgebracht werden. Als Templatpartikel werden dabei insbesondere teilvernetzte Melaminformaldehydpartikel beschrieben. Nach Bildung der Poylelektrolythülle können die Melaminformaldehydpartikel durch Einstellung eines sauren pH-Werts oder durch Sulfonierung aufgelöst werden.

Überraschenderweise wurde gefunden, daß Polyelektrolytkapseln auch unter Verwendung von Templaten ausgewählt aus biologischen Zellen, Aggregaten biologischer oder/und amphiphiler Materialien wie etwa Erythrozyten, Bakterienzellen oder Lipidvesikeln gebildet werden können. Die eingekapselten Templatpartikel können durch anschließende Solubilisierung bzw. Desintegration entfernt werden.

Die Erfindung betrifft somit ein Verfahren zur Herstellung von Kapseln mit einer Polyelektrolythülle, die einen Durchmesser im Bereich von 10 nm bis 10 µm aufweisen wobei man auf einem Templat ausgewählt aus Aggregaten biologischer oder/und amphiphiler Materialien mehrere aufeinanderfolgende Schichten entgegengesetzt geladener Polyelektrolytmoleküle aufbringt, indem man zunächst eine Dispersion der Templatpartikel in einer wässrigen Lösung erzeugt, zu dieser Dispersion dann eine Polyelektrolytspezies mit der gleichen oder der entgegengesetzten Ladung wie die Oberfläche des Templatpartikels gibt, anschließend nach Abtrennung gegebenenfalls vorhandener überschüssiger Polyelektrolytmoleküle die für den Aufbau der zweiten Schicht verwendete entgegengesetzt geladene Polyelektrolytspezies zugibt und dann gegebenenfalls weitere abwechselnd entgegengesetzt geladene Schichten von Polyelektrolytmolekülen aufgebracht werden, und gegebenenfalls anschließend das Templat desintegriert.

Als Templatmaterialien können beispielsweise Zellen, z.B. eukaryontische Zellen, wie etwa Säugererythrozyten oder Pflanzenzellen, einzellige Organismen wie etwa Hefen, Bakterienzellen wie etwa E.coli Zellen, Zellaggregate, subzelluläre Partikel wie etwa Zellorganellen, Pollen, Membranpräparationen oder Zellkerne, Viruspartikel und Aggregate von Biomolekülen, z.B. Proteinaggregate wie etwa Immunkomplexe, kondensierte Nukleinsäuren, Ligand-Rezeptor-Komplexe etc. verwendet werden. Das erfindungsgemäße Verfahren eignet sich auch zur Verkapselung lebender biologischer Zellen und Organismen. Ebenso als Template geeignet sind Aggregate amphiphiler Materialien, insbesondere Membranstrukturen wie etwa Vesikel, z.B. Liposomen oder Micellen sowie andere Lipidaggregate.

Auf diese Template werden mehrere entgegengesetzt geladene Polyelektrolytschichten abgeschieden. Hierzu werden die Templatpartikel vorzugsweise zunächst in einem geeigneten Lösungsmittel, z.B. einem wässrigen Medium dispergiert. Dann kann - insbesondere wenn es sich bei den Templatpartikeln um Zellen oder andere biologische Aggregate handelt - ein Fixierungsreagenz in ausreichender Konzentration zugesetzt werden, um eine mindestens partielle Fixierung der Templatpartikel zu bewirken. Beispiele für Fixierungsreagenzien sind Aldehyde wie Formaldehyd oder Glutardialdehyd, die vorzugsweise dem Medium auf eine Endkonzentration zwischen 0,1 - 5% (w/w) zugesetzt werden.

Unter Polyelektrolyten werden allgemein Polymere mit ionisch dissoziierbaren Gruppen, die Bestandteil oder Substituent der Polymerkette sein können, verstanden. Üblicherweise ist die Zahl dieser ionisch dissoziierbaren Gruppen in Polyelektrolyten so groß, daß die Polymeren in der dissoziierten Form (auch Polyionen genannt) wasserlöslich sind. Hierin werden unter dem Begriff Polyelektrolyte auch lonomere verstanden, bei denen die Konzentration der ionischen Gruppen für eine Wasserlöslichkeit nicht ausreichend sind, die jedoch genügend Ladungen aufweisen, um eine Selbstassemblierung einzugehen. Bevorzugt umfasst die Hülle "echte" Polyelektrolyte. Je nach Art der dissoziierbaren Gruppen werden Polyelektrolyte in Polysäuren und Polybasen unterteilt.

Aus Polysäuren entstehen bei der Dissoziation unter Abspaltung von Protonen Polyanionen, die sowohl anorganische als auch organische Polymere sein können. Beispiele für Polysäuren sind Polyphosphorsäure, Polyvinylschwefelsäure, Polyvinylsulfonsäure, Polyvinylphosphonsäureund Polyacrylsäure. Beispiele für die entsprechenden Salze, die auch als Polysalze bezeichnet werden, sind Polyphosphat, Polysulfat, Polysulfonat, Polyphosphonat und Polyacrylat.

Polybasen enthalten Gruppen, die in der Lage sind, Protonen, z.B. durch Reaktion mit Säuren unter Salzbildung, aufzunehmen. Beispiele für Polybasen mit ketten - bzw. seitenständigen dissoziierbaren Gruppen sind Polyallylamin, Polyethylenimin, Polyvinylamin und Polyvinylpyridin. Polybasen bilden durch Aufnahme von Protonen Polykationen.

Erfindungsgemäß geeignete Polyelektrolyte sind sowohl Biopolymere, wie etwa Alginsäure, Gummi arabicum, Nucleinsäuren, Pektine, Proteine und andere, sowie chemisch modifizierte Biopolymere, wie etwa Carboxymethylcellulose und Ligninsulfonate sowie synthetische Polymere, wie etwa Polymethacrylsäure, Polyvinylsulfonsäure, Polyvinylphosphonsäure und Polyethylenimin.

Es können lineare oder verzweigte Polyelektrolyte eingesetzt werden. Die Verwendung verzweigter Polyelektrolyte führt zu weniger kompakten Polyelektrolytmultifilmen mit einem höheren Grad der Wandporosität. Zur Erhöhung der Kapselstabilität können Polyelektrolytmoleküle innerhalb oder/und zwischen den einzelnen Schichten vernetzt werden, z.B. durch Crosslinking von Aminogruppen mit Aldehyden. Weiterhin können amphiphile Polyelektrolyte, z.B. amphiphile Block- oder Randomcopolymere mit partiellem Polyelektrolytcharakter zur Verringering der Permeabilität gegenüber polaren kleinen Molekülen eingesetzt werden. Solche amphiphilen Copolymere bestehen aus Einheiten unterschiedlicher Funktionalität, z.B. einerseits sauren oder basischen Einheiten und andererseits hydrophoben Einheiten wie Styrolen, Dienen oder Siloxanen etc. die als Blöcke oder statistisch verteilt über das Polymer angeordnet sein können. Durch Verwendung von Copolymeren, die als Funktion äußerer Bedingungen ihre Struktur ändern, können die Kapselwände bezüglich ihrer Permeabilität oder anderer Eigenschaften definiert gesteuert werden. Hierzu bieten sich beispielsweise Copolymere mit einem Poly(N-isopropyl-acrylamid)-Anteil, z.B. Poly(N-isopropylacrylamid-acrylsäure) an, die über das Gleichgewicht von Wasserstoffbrückenbindungen ihre Wasserlöslichkeit als Funktion der Temperatur ändern, was mit einer Quellung einhergeht.

Durch Verwendung von unter bestimmten Bedingungen abbaubaren, z.B. photo-, säure- oder baselabilen Polyelektrolyten kann über die Auflösung der Kapselwände die Freisetzung von eingeschlossenen Wirkstoffen gesteuert werden. Weiterhin können für bestimmte Anwendungsmöglichkeiten auch leitende Polyelektrolyten oder Polyelektrolyten mit optisch aktiven Gruppen als Kapselkomponenten verwendet werden.

Grundsätzlich ergeben sich keine Einschränkungen hinsichtlich der zu verwendenden Polyelektrolyte bzw. lonomere, solange die verwendeten Moleküle eine genügend hohe Ladung aufweisen oder/und die Fähigkeit besitzen, über andere Wechselwirkungsarten, wie beispielsweise Wasserstoffbrückenbindungen und/oder hydrophobe Wechselwirkungen, eine Bindung mit der darunter liegenden Schicht einzugehen.

Geeignete Polyelektrolyte sind somit sowohl niedermolekulare Polyelektrolyte bzw. Polyionen als auch makromolekulare Polyelektrolyte, beispielsweise auch Polyelektrolyte biologischer Herkunft.

Zum Aufbringen der Polyelektrolytschichten auf das Templat wird vorzugsweise zunächst eine Dispersion der Templatpartikel in einer wässrigen Lösung erzeugt. Zu dieser Dispersion gibt man dann eine Polyelektrolytspezies mit der gleichen oder der entgegengesetzten Ladung wie die Oberfläche des Templatpartikels. Nach Abtrennung evtl. vorhandender überschüssiger Polyelektrolytmoleküle wird die für den Aufbau der zweiten Schicht verwendete entgegengesetzt geladene Polyelektrolytspezies zugegeben. Anschließend werden weiterhin abwechselnd entgegengesetzt geladene Schichten von Polyelektrolytmolekülen aufgebracht, wobei für jede Schicht mit gleicher Ladung gleiche oder verschiedene Polyelektrolytspezies oder Gemische von Polyelektrolytspezies gewählt werden können. Die Anzahl von Schichten kann grundsätzlich beliebig gewählt werden und beträgt beispielsweise 2 bis 40, insbesondere 4 bis 20 Polyelektrolytschichten.

Nachdem die gewünschte Anzahl Schichten aufgebracht worden ist, können - sofern gewünscht - die nun umhüllten Templatpartikel desintegriert werden. Die Desintegration kann durch Zugabe von Lysereagenzien erfolgen. Dabei sind Lysereagenzien geeignet, die biologische Materialien wie Proteine oder/und Lipide auflösen können. Vorzugsweise enthalten die Lysereagenzien ein Deproteinisierungsmittel, beispielsweise Peroxoverbindungen wie etwa H₂O₂ oder/und Hypochloritverbindungen wie etwa Natrium- oder Kaliumhypochlorit. Überraschenderweise erfolgt die Desintegration der Templatpartikel innerhalb einer kurzen Inkubationsdauer, z.B. 1 min bis 1 h bei Raumtemperatur. Die Desintegration der Templatpartikel ist weitgehend vollständig, da selbst bei elektronenmikroskopischer Betrachtung der verbleibenden Hüllen keine Reste der Partikel mehr nachweisbar sind. Bei Einbau biologischer Polyelektrolyten in die Hülle können leere Schichten auch innerhalb der Polyelektrolythülle erzeugt werden.

Die Erfindung betrifft weiterhin Polyelektrolyt-Kapseln, die durch das erfindungsgemäße Verfahren erhältlich sind, und die Templatpartikel in ihrem Inneren enthalten. Leere Kapseln, die keine Templatpartikel mehr enthalten, sind nicht Gegenstand der Erfindung.

Durch das erfindungsgemäße Verfahren erhältliche Kapseln können mit Durchmessern im Bereich von 10 nm bis 10 µm auch in der von der Kugelgestalt abweichenden, d.h. in anisotropen Formen hergestellt werden. Die Wandstärke wird durch die Anzahl der Polyelektrolytschichten bestimmt und kann beispielsweise im Bereich von 2 bis 100 nm, insbesondere im Bereich von 5 bis 80 nm liegen. Die Kapseln zeichnen sich auch durch ihre Monodispersität aus, d.h. bei Auswahl geeigneter Template können Kapselzusammensetzungen erhalten werden, bei denen der Anteil an Kapseln, deren Abweichung vom mittleren Durchmesser > 50% ist, weniger als 10% und besonders bevorzugt weniger als 1 % beträgt.

Die Kapseln sind gegenüber chemischen, biologischen, organischen und thermischen Belastungen sehr stabil. Sie können eingefroren oder gefriergetrocknet und anschließend wieder in geeigneten Lösungsmitteln aufgenommen werden.

Da die Kapseln Mikroabdrücke der in ihnen enthaltenen Template darstellen und auch nach Entfernung der Template ihre Form beibehalten, können anisotrope Partikel hergestellt werden, bei denen es sich um Mikroabdrücke von biologischen Strukturen wie Zellen, Viruspartikeln oder Biomolekülaggregaten handelt.

Eine Modifizierung der Permeabilitätseigenschaften in der Hülle kann durch Bildung oder Veränderung von Poren in mindestens einer der Polyelektrolytschichten erreicht werden. Solche Poren können bei Verwendung entsprechender Polyelektrolyten selbst gebildet werden. Weiterhin können Nanopartikel mit anionischen oder/und kationischen Gruppen oder/und grenzflächenaktive Substanzen wie etwa Tenside oder/und Lipide zur Modifizierung der Permeabilität und anderer Eigenschaften eingesetzt werden. Darüber hinaus kann die Permeabilität durch Variation der Bedingungen, die beim Abscheiden des Polyelektrolyten herrschen, modifiziert werden. So führt beispielsweise eine hohe Salzkonzentration des Umgebungsmediums zu einer hohen Durchlässigkeit der Polyelektrolythülle.

Eine besonders bevorzugte Modifizierung der Permeabilität von Polyelektrolythüllen kann durch Abscheidung von Lipidschichten oder/und amphiphiler Polyelektrolyten auf der Polyelektrolythülle nach Desintegration der Templatpartikel erreicht werden. Auf diese Weise kann die Permeabilität der Polyelektrolythüllen für kleine und polare Moleküle sehr stark vermindert werden. Beispiele für Lipide, die auf den Polyelektrolythüllen abgeschieden werden können, sind Lipide, die mindestens eine ionische oder ionisierbare Gruppe tragen, z.B. Phospholipide wie etwa Dipalmitoylphosphatidinsäure oder zwitterionische Phospholipide wie etwa Dipalmitoylphosphatidylcholin oder auch Fettsäuren bzw. entsprechende langkettige Alkylsulfonsäuren. Bei Verwendung zwitterionischer Lipide können Lipidmultischichten auf der Polyelektrolythülle abgeschieden werden. Auf den Lipidschichten können anschließend weitere Polyelektrolytschichten abgeschieden werden.

Die durch das Verfahren erzeugten Kapseln können zum Einschluß von Wirkstoffen verwendet werden. Diese Wirkstoffe können sowohl anorganische als auch organische Stoffe sein. Beispiele für solche Wirkstoffe sind Katalysatoren, insbesondere Enzyme, pharmazeutische Wirkstoffe, Polymere, Farbstoffe wie etwa fluoreszierende Verbindungen, Sensormoleküle, d.h. Moleküle die auf die Änderung von Umgebungsbedingungen (Temperatur, pH-Wert) nachweisbar reagieren, Pflanzenschutzmittel und Aromastoffe.

Die Kapseln können auch als Mikroreaktionsräume für chemische Reaktionen oder als Präzipitations- oder Kristallisationstemplate verwendet werden. Aufgrund der Tatsache, daß die Permeabilität der Kapselwände steuerbar ist, so daß sie beispielsweise niedermolekulare Substanzen passieren lassen, Makromoleküle jedoch weitgehend zurückhalten, lassen sich bei einer chemischen Reaktion entstehende hochmolekulare Produkte, z.B. bei einer Polymerisation entstehende Polymere auf einfache Weise während der Synthese im Innenraum zurückhalten. Das gleichzeitig im Außenmedium synthetisierte Reaktionsprodukt kann z.B. durch Zentrifugation oder/und Filtration nachträglich oder auch bereits während der Reaktion entfernt werden.

Während der Reaktion kann die Zufuhr des Reaktionssubstrats über die Diffusion durch die Kapselwände gesteuert werden. Dabei ergeben sich neue Wege, in Reaktionsabläufe einzugreifen. Da z.B. durch Filtration ein kontinuierlicher oder z.B. durch Zentrifugation auch ein plötzlicher Austausch des Außenmediums möglich ist, kann die Polymerisationsreaktion durch Substratentfernung beliebig angehalten werden bzw. das Monomer kann ausgetauscht werden. Es ist somit möglich, auf neue Art und Weise die Herstellung von definierten Co- oder Multipolymeren durchzuführen. Da durch die Permeation der Reaktionsablauf über die Monomerenzufuhr kontrollierbar ist, können in den Kapseln Produkte mit neuen und anderen Molekulargewichtsverteilungen, z.B. hoch monodisperse Produkte erzeugt werden. Im Kapselinneren synthetisierte Polymere lassen sich z.B. durch Titration mit Fluoreszenzfarbstoffen spektroskopisch und durch konvokale Mikroskopie nachweisen. Mit Einzelteilchenlichtstreuung läßt sich der Massenzuwachs und somit die Reaktionskinetik verfolgen.

Bei Verwendung anisotroper Kapseln zur Verpackung von Wirkstoffen oder als Reaktionsräume, z.B. für Synthesen oder Präzipitationsprozesse, und gegebenenfalls anschließender Auflösung der Templathüllen können Partikelzusammensetzungen als Dispersionen mit vorbestimmten Formen und Gestalten erzeugt werden. Die Erfindung betrifft somit auch anisotrope Partikelzusammensetzungen, die durch Verkapselung von Wirkstoffen in einer Polyelektrolythülle, z.B. durch Synthese oder Präzipitation und anschließende Entfernung des Templats, z.B. durch thermische oder chemische Behandlung, erhältlich sind. Vorzugsweise besitzen diese anisotropen Partikel die Form der als Templat verwendeten Biostrukturen.

Weiterhin können die Kapseln zum Einbringen von organischen Flüssigkeiten wie etwa Alkoholen oder Kohlenwasserstoffen, z.B. Hexanol, Octanol, Octan oder Decan, oder zum Verkapseln von Gasen verwendet werden. Solche mit einer organischen, nicht mit Wasser mischbaren Flüssigkeit gefüllten Kapseln können auch für chemische Reaktionen, z.B. Polymerisationsreaktionen eingesetzt werden. So kann das Monomer über dessen Verteilungsgleichgewicht gezielt im Innenraum der Kapseln angereichert werden. Gegebenenfalls kann die Monomerenlösung bereits vor Beginn der Synthese im Innenraum eingekapselt werden.

Es können jedoch auch Wirkstoffe verkapselt werden, die aufgrund ihrer Größe nicht die Polyelektrolythülle durchdringen können. Hierzu wird der einzuschließende Wirkstoff an das Templatpartikel immobilisiert oder vom Templatpartikel eingekapselt, z.B. durch Phagozytose oder Endozytose bei lebenden Zellen. Nach Desintegration der Templatpartikel wird der Wirkstoff ins Innere der Polyelektrolythülle freigesetzt. Dabei werden zweckmäßigerweise die Bedingungen bei der Desintegration des Templatpartikels so gewählt, daß keine unerwünschte Zersetzung des Wirkstoffs eintritt.

Die Kapseln können auf zahlreichen Anwendungsgebieten, beispielsweise Sensorik, Oberflächenanalytik, als Emulsionsträger, Mikroreaktionsräume wie etwa für katalytische Prozesse, Polymerisation-, Präzipitations- oder Kristallisationsprozesse, in der Pharmazie und Medizin, z.B. zum Targeting von Wirkstoffen oder als Ultraschallkontrastmittel, in der Lebensmitteltechnologie, Kosmetik, Biotechnologie, Sensorik, Informationstechnologie und Druckindustrie (Einkapselung von Farbstoffen) eingesetzt werden. Weiterhin können die Kapseln zum Aufbau von Mikro- bzw. Nanokompositen, d.h. Werkstoffen, die aus mindestens zwei verschiedenen Materialien bestehen und eine Mikro- bzw. nanoskopische Ordnung aufweisen, eingesetzt werden.

Noch ein weiterer Aspekt der Erfindung besteht darin, die Templatpartikel vorzugsweise in fixierter Form vor der Polyelektrolytbeschichtung durch Behandlung mit einem Lysereagenz partiell zu desintegrieren. Bei rechtzeitiger Unterbrechung des Lyseprozesses erhält man partiell aufgelöste Strukturen, z.B. toroidale Strukturen mit einem Loch in der Mitte, die anschließend beschichtet werden können. Nach anschließendem vollständigen Abbau der Templatpartikel erhält man als Resultat ringförmige Kapseln. Dies ist eine völlig neue topologische Qualität mit interessanten Anwendungsmöglichkeiten, z.B. in der Optik (Mikroflüsterbogeneffekt).

Die Erfindung wird durch die nachfolgenden Beispiele und Figuren weiter erläutert. Es zeigen:
- Figur 1: die Änderungen des Zeta potentials (A) und den Anstieg der Fluoreszenzintensität (B) als Funktion der Schichtenanzahl für die Abscheidung von Poly(styrolsulfonat-Natriumsalz) (PSS) und Poly(allylaminhydrochlorid) (PAH) auf mit Glutardialdehyd fixierten humanen Erythrozyten.
- Figur 2: ein rasterelektronenmikroskopisches Bild eines mit zehn Schichten PSS und PAH bedeckten Discozyten.
- Figur 3: transmissionselektronenmikroskopische Abbildungen eines unbeschichteten (A) und eines beschichteten (B) Discozyten sowie einer nach Auflösung des beschichteten Discozyten erhaltenden Polyelektrolythülle (C).
- Figur 4: atomkraftmikroskopische Abbildungen von auf Discozyten (A) und Echinozyten (B) abgeschiedenen Polyelektrolythüllen.
- Figur 5: mit einem konfokalen Mikroskop aufgenommene Bilder von auf Echinozyten abgeschiedenen und aus 11 Schichten PSS/PAH bestehenden Polyelektrolythüllen.
- Figur 6: mit einem konfokalen Mikroskop aufgenommene Bilder von mit 6-Carboxyfluorescein gefüllten Polyelektrolythüllen.
- Figur 7: Elektrorotationsspektren von Polyelektrolythüllen auf Discozyten ohne zusätzliche Beschichtung (A) oder beschichtet mit DPPA (B) oder DPPC (C).
- Figur 8: die Abbildung einer auf einem Discozyten abgeschiedenen Polyelektrolythülle in einem Lichtmikroskop (A) und das entsprechende rasterelektronenmikroskopische Bild (B).
- Figur 9: die Bildung von Poly(diallylmethylammoniumchlorid) durch radikalische Polymerisation auf der Außenseite und im Inneren von Polyelektrolythüllen.

### Beispiele

### 1. Präparation von Polymerhüllen mit Rinder- oder Humanerythrozyten als Templat

Von frischem Human- oder Rinderblut wird das Plasma abzentrifugiert. Anschließend folgt zweimaliges Waschen in einer isotonen Phosphatgepufferten Kochsalzlösung PBS (5,8 mM Phosphatpuffer pH 7,4, KCI 5,6 mM, NaCl 150 mM). Anschließend werden die Erythrozyten mit Glutardialdehyd in einer Konzentration von 2% fixiert. Dazu wird 1 ml des Erythrozytensediments mit 1 ml PBS aufgefüllt. Zu dieser Lösung werden dann tröpfchenweise 8 ml einer Glutardialdehydlösung (1 Teil Glutardialdehyd (25%ige wässrige Lösung) und 9 Teile PBS) zugegeben. Nach 60 min Einwirkzeit bei 20°C wird die Lösung abzentrifugiert und die Erythrozyten werden viermal in bidestillierten Wasser gewaschen. Anschließend werden die fixierte Erythrozyten mit ungepufferter 154 mM NaCl Lösung aufgefüllt.

Als nächster Schritt folgt die konsekutive Adsorption von zwei entgegengesetzt geladenen Polyelektrolyten. Da die Ausgangsladung der fixierten Erythrozyten negativ ist, wird vorzugsweise zunächst positiv geladenes Poly(allylamin)hydrochlorid (PAH) mit einem Molekulargewicht zwischen 50 und 60 kD (Aldrich) verwendet. Es kann jedoch auch ein negativ geladener Polyelektrolyt als erste Schicht auf den Erythrozyten abgeschieden werden. Zur Beschichtung der Erythrozyten werden 4 ml Lösung mit einer Konzentration von 0,5 g/dl PAH und 0,5 M NaCl bei einer Erythrozytenkonzentration von ca. 2,5 (v/v) angesetzt. Nach 10 min Einwirkzeit bei 20°C werden die Erythrozyten abzentrifugiert und zweimal in einer 154 mM NaCl Lösung gewaschen. Anschließend folgt die Adsorption der zweiten Schicht. Zu diesem Zweck wird negativ geladenes Poly(styrolsulfonat)-Natriumsalz (PSS) mit einem Molekulargewicht von 70 kD verwendet. Zum Aufbringen der ersten PSS-Schicht auf die bereits mit PAH beschichteten Erythrozyten werden 4 ml Lösung mit einer Konzentration von 0,5 g/dl PSS und 0,5 M NaCl und einer Erythrozytenkonzentration von ca. 2,5% (v/v) angesetzt. Nach 10 min Einwirkzeit bei 20°C werden die Erythrozyten abzentrifugiert und zweimal in einer 154 mM NaCl Lösung gewaschen. Das Aufbringen von PAH- und PSS-Schichten kann beliebig oft wiederholt werden. Beispielsweise können jeweils 5 PAH- und 5 PSS-Schichten aufgebracht werden.

Zur Auflösung des Templats werden die fixierten Erythrozyten in eine 1,2%ige NaOCl Lösung pipettiert. Ebenso geeignet sind handelsübliche Deproteinizer (Produkt, Hersteller) oder Abflußreiniger (z.B. Chlorix, Hersteller). Die Einwirkzeit beträgt ca. 20 min bei 20°C und läßt sich optisch über die verschwindende Trübung der Lösung kontrollieren. Die verbleibenden Polymerhüllen werden anschließend in NaCl Lösung gewaschen.

### 2. Präparation von Polymerhüllen mit E.coli Bakterien oder Hefen als Templat

Zunächst werden die E.coli Zellen durch zweimaliges Waschen in einer isotonen PBS-Lösung vom Nährmedium getrennt. Anschließend erfolgt die Fixierung mit Glutardialdehyd. Hierzu wird das Sediment der Coli-Bakterien auf 2 ml mit PBS aufgefüllt. Zu dieser Lösung werden 8 ml einer Glutardialdehydlösung auf eine Endkonzentration von 2% zugegeben. Nach 60 min Einwirkzeit bei 20°C wird die Lösung abzentrifugiert und die fixierten E.coli Zellen werden viermal in bidestilliertem Wasser gewaschen.

Anschließend folgt eine konsekutive Adsorption von zwei entgegengesetzt geladenen Polyelektrolyten wie in Beispiel 1 beschrieben.

Auf entsprechende Weise wurden - ohne vorherige Fixierung - auch Hefezellen beschichtet.

### 3. Abscheidung von Lipidschichten auf Polyelektrolythüllen

Zur Abscheidung von Lipidschichten auf Polyelektrolythüllen wurden zwei unterschiedliche Verfahren verwendet.

### 3.1

200 µl einer Suspension von Polyelektrolythüllen werden durch wiederholtes Waschen in Methanol resuspendiert. Nach dem dritten Waschen werden anstelle von reinem Methanol 500 µl einer Lipidlösung von z.B. 1 mg/ml Dipalmitoylphosphatidinsäure (DPPA) oder Dipalmitoylphosphatidylcholin (DPPC) in Methanol dem Sediment zugesetzt. Die Hüllen werden in dieser Methanol-Lipidlösung resuspendiert und die Suspension bei einer Temperatur von 90°C in einem Wasserbad gehalten. Der verdampfende Methanol wird durch tropfenweise Zugabe von Wasser in Portionen von jeweils 20 µl ersetzt. Der Austausch von 700 µl Methanol gegen Wasser dauert etwa 30 min.

Nach Beendigung der Verdampfung wird die Hüllensuspension dreimal mit Wasser gewaschen und wiederholt zentrifugiert. Durch 20 min Zentrifugation bei 25 000 Upm können die Lipid-beschichteten Hüllen sedimentiert werden.

### 3.2

Dispersionen von DPPA oder 90% DPPC und 10% DPPA mit einer Konzentration von 1 mg Lipid/ml in Wasser werden durch Ultraschallbehandlung hergestellt. 500 µl der resultierenden Dispersion von Lipidvesikeln werden auf 200 µl einer konzentrierten Hüllensuspension gegeben. Nach 30 min werden die Proben 20 min bei 25 000 Upm zentrifugiert. Der Überstand wird entfernt und durch Wasser ersetzt. Diese Prozedur wird dreimal wiederholt. Dabei wird eine konzentrierte Suspension von Lipid-beschichteten Hüllen erhalten.

### 4. Einschluß von organischen Lösungsmitteln in Polyelektrolythüllen

Eine wässrige Suspension von Polyelektrolythüllen wird 5 min bei 3000 Upm zentrifugiert. Nach Entfernen des Überstands wird Methanol zugegeben. Die Hüllen werden resuspendiert und 10 min lang bei 4000 Upm zentrifugiert. Erneut wird der Überstand entfernt, Methanol zugegeben und die Probe unter denselben Bedingungen wie zuvor zentrifugiert. Diese Prozedur wird dreimal wiederholt. Nach der letzten Zentrifugation mit Methanol wird der Überstand durch Hexanol ersetzt. Die Hüllen werden resuspendiert und 10 min bei 5000 Upm zentrifugiert. Diese Prozedur wird wiederum dreimal wiederholt.

Zum Einschluß von Octanol, Octan oder Decan in die Hüllen wird eine ähnliche Prozedur verwendet, wobei als Ausgangsmaterial die in einer Hexanollösung vorliegenden Hüllen verwendet werden. Die Zentrifugationsgeschwindigkeit wird für Octanol und Octan auf 7000 Upm (10min) und für Decan auf 7500 Upm (10min) erhöht.

Schließlich wird das resultierende Sediment in Wasser resuspendiert. Die Hüllen verbleiben in der wässrigen Phase, während die noch vorhandenen Spuren des Lösungsmittels im Sediment zwischen den Hüllen eine zweite organische Phase bilden. Durch Verwendung von Fluoreszenzmarkern für die organische und die wässrige Phase kann mittels konfokaler Mikroskopie gezeigt werden, daß die Hüllen mit organischem Lösungsmittel gefüllt sind.

Die beschriebene Prozedur ermöglicht die Herstellung einer hochstabilen Emulsion von nichtpolaren Flüssigkeiten in Wasser. Als Folge der Monodispersität der ursprünglichen Hüllen ist die erzeugte Emulsion ebenso monodispers. Ein weiterer Vorteil ist, daß selbst die Form der einzelnen Tröpfchen - abhängig vom verwendeten Templat - reguliert werden kann. Dies ermöglicht die Herstellung von Emulsionen mit Oberfläche:Volumen-Verhältnissen, die von denjenigen einer Kugel verschieden sind.

### 5. Charakterisierung von Polyelektrolythüllen

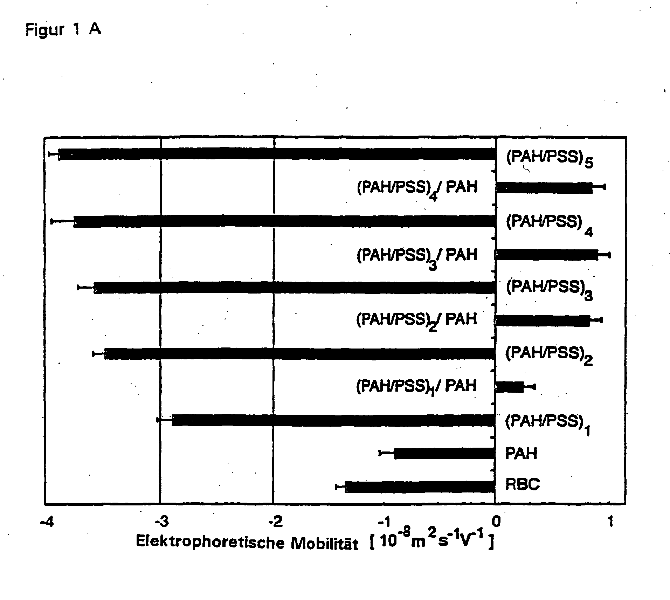

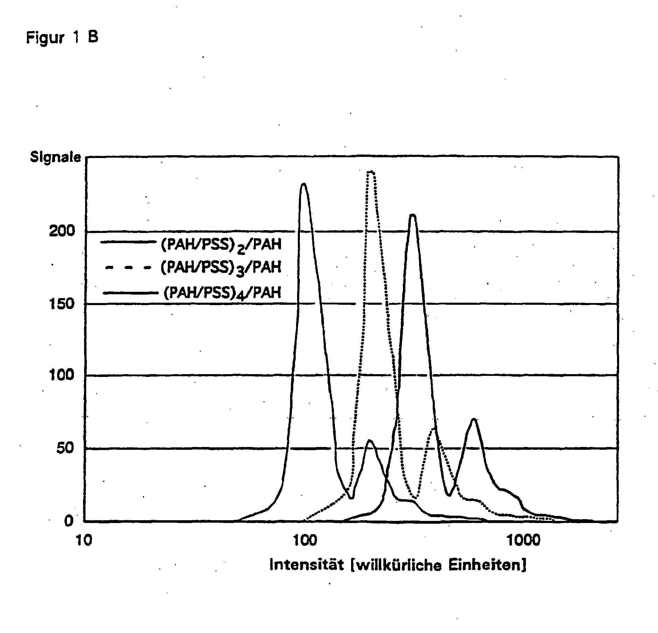

Figur 1 zeigt die Änderungen im Zetapotential (A) und den Anstieg der Fluoreszenzintensität (B) als Funktion der Schichtenzahl bei der Abscheidung von Poly(styrolsulfonat-Natriumsalz) und Poly(allylaminhydrochlorid) auf mit Glutardialdehyd vorbehandelten humanen Erythrozyten. Das Zetapotential wird durch elektrophoretische Mobilitätsmessungen (Elektrophor, Hasotec) in physiologischer Salzlösung bestimmt. Die Fluoreszenzintensitätsverteilungen werden durchflußzytometrisch (FACScan, Becton Dickinson) unter Verwendung von FITC-markiertem PAH in drei aufeinanderfolgenden Schichtabscheidungszyklen aufgezeichnet.

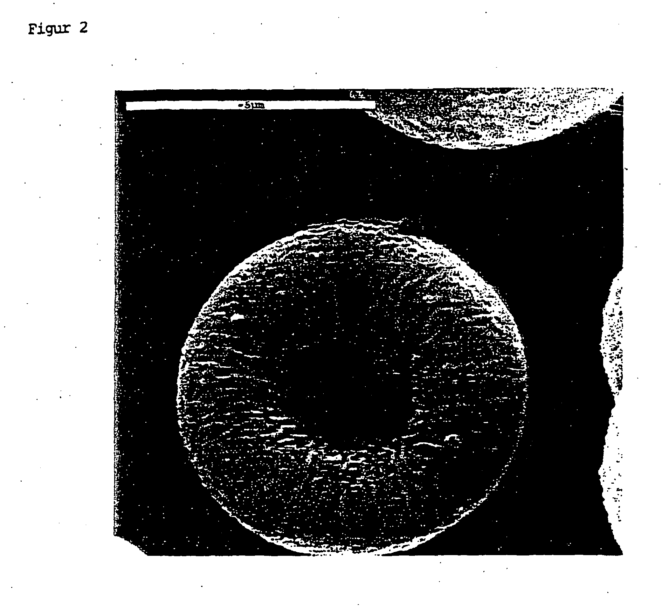

In Figur 2 ist das rasterelektronenmikroskopische Bild eines mit zehn Schichten von PSS und PAH bedeckten Discozyten gezeigt. Der Trocknungsprozeß führt zum Enstehen longitudinaler Falten der Polyelektrolytschicht entlang dem Rand der Zelle.

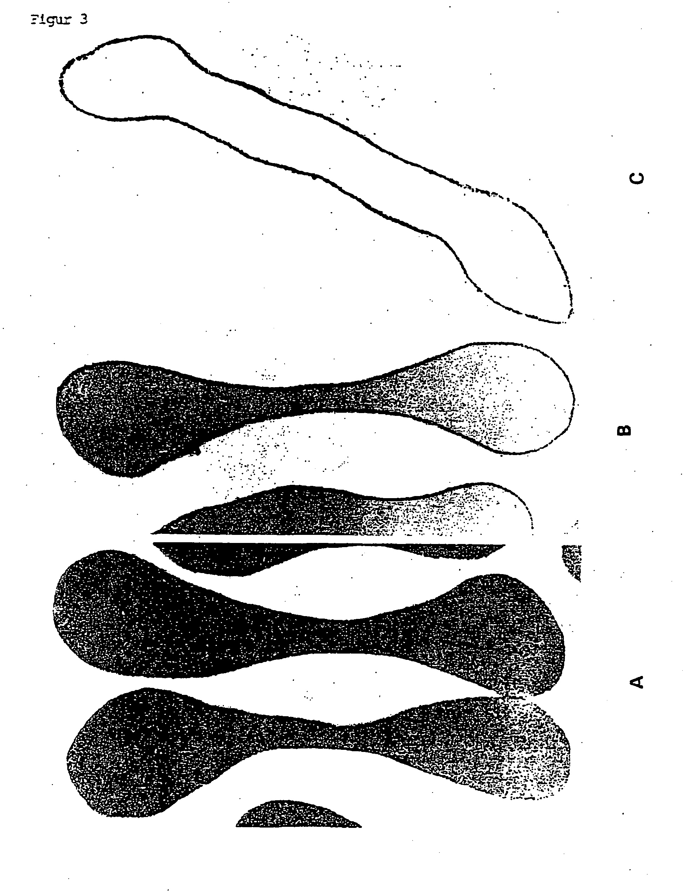

Figur 3 zeigt transmissionselektronenmikroskopische Bilder von unbeschichteten (A) und beschichteten (B) Discozyten. Die Polyelektroly-hülle ist deutlich zu erkennen. Die nach Solubilisierung der Zelle erhaltene Polyelektrolythülle (C) zeigt zwei überraschende Eigenschaften. Die Vergrößerungen sind 1:15000 bei A und B und 1:17000 bei C. Erstens ist sie der Form der ursprünglichen Zelle ähnlich und zweitens scheint sie vollkommen leer zu sein, ohne daß Risse oder größere Poren in der Hülle zu erkennen sind.

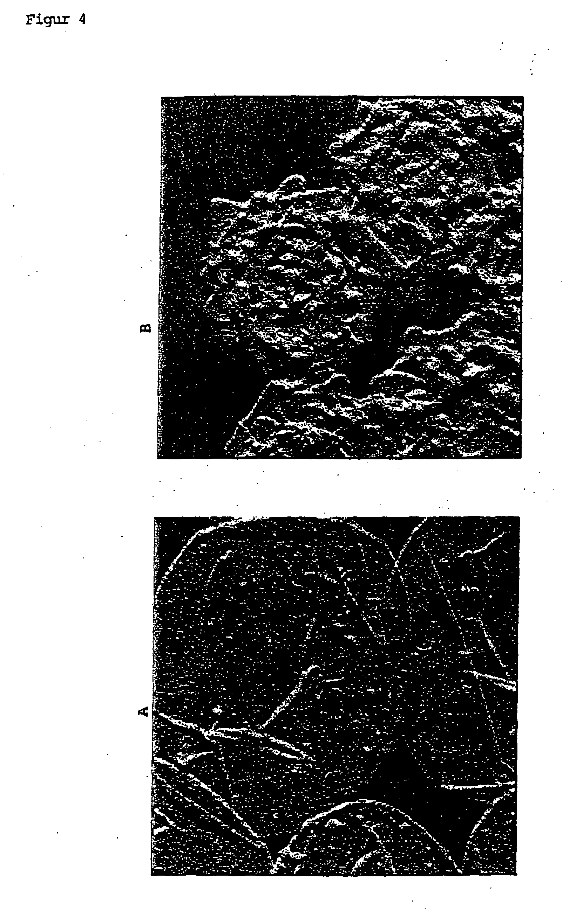

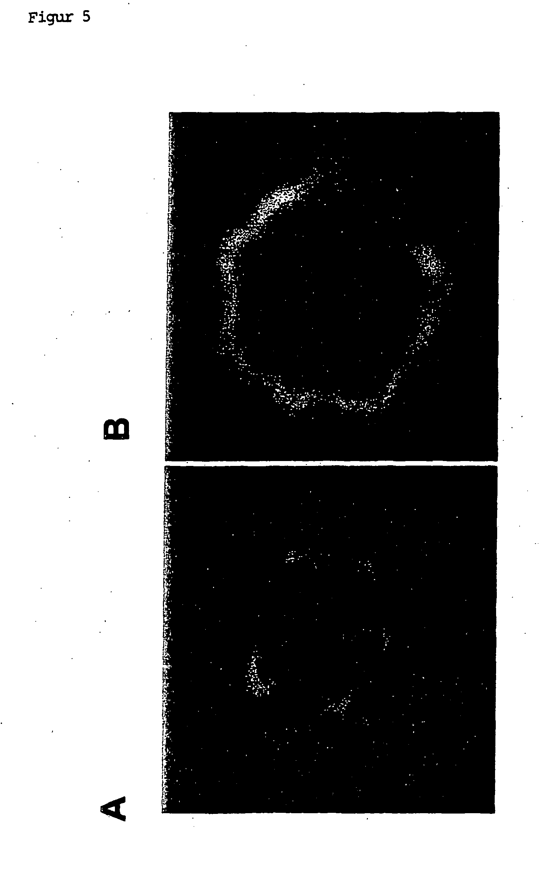

In Figur 4 sind zwei mit Atomkraft-Mikroskopie (AFM) erzeugte Bilder (Breite 10 µm) dargestellt, die auf einem Discozyten (A) und einem Echinozyten (B) abgeschiedene Polyelektrolythüllen aus insgesamt 9 Schichten zeigen. Während die auf einem ellipsoidalen Discozyten abschiedenen Hüllen nur wenige Falten zeigen, führt der Abscheideprozeß auf einem sternartigen Echinozyten zu gut strukturierten Hüllen, auf denen sogar die Vorsprünge des ursprünglichen Templats zu erkennen sind. Dies geht noch deutlicher aus den in Figur 5 gezeigten konfokalen Mikroskopbildern einer auf einem Echinozyten abgeschiedenen Polyelektrolythülle aus elf Schichten PSS/PAH hervor. Die äußere Schicht besteht aus mit FITC markiertem PAH. Die Breite der Bilder ist 7 µm. Die Scans wurden in 2 Ebenen getrennt durch einen Abstand von 1 µm gemacht. Scan A läuft durch den oberen Teil der auf einem Glasträger aufgebrachten Hülle. Auf diesen Bildern ist zu erkennen, daß das Innere der Hülle, selbst im Bereich der Vorsprünge leer ist.

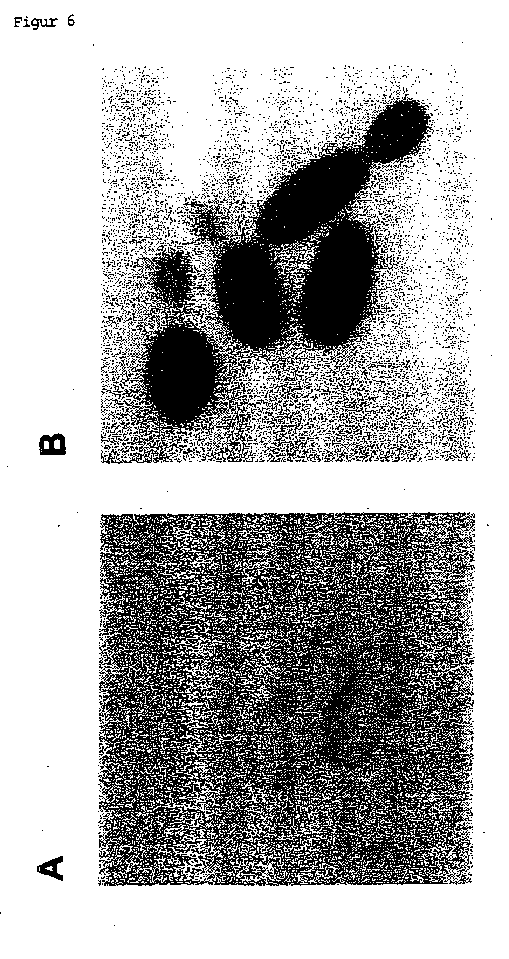

Figur 6 zeigt mit einem konfokalen Mikroskop aufgenommene Bilder von auf Discozyten abgeschiedenen Polyelektrolythüllen, die aus 10 Schichten PSS/PAH bestehen. Die Hüllen wurden mit einer 100 µM 6-Carboxyfluorescein (6-CF) Lösung behandelt. In Figur 6 A ist eine Fluoreszenz innerhalb der Hüllen zu erkennen. Dies zeigt, daß die 6-CF Moleküle in das Innere der Hülle eindringen können.

Bei Inkubation der Hüllen mit 100 nM 6-CF konnte keine Fluoreszenz gefunden werden. Dies zeigt, daß durch Behandlung mit dem Lysereagenz die zur Bindung von 6-CF fähigen Aminogruppen von PAH abgebaut oder/und blockiert werden. Aufgrund der geringen Konzentration von 6-CF ist die Lösungsfluoreszenz zu gering, um als Hintergrund nachgewiesen werden zu können.

Durch Adsorption von Dipalmitoylphosphatidinsäure (DPPA) oder zwitterionischen Lipiden beispielsweise Dipalmitoylphosphatidylcholin (DPPC) auf Polyelektrolythüllen gemäß Beispiel 3 durch Verdampfen werden mit stabilen Lipidschichten bedeckte Polyelektrolythüllen erhalten. Die Lipidschicht verhindert weitgehend das Eindringen von 6-CF in die Hülle (Figur 6 B). Die Breite der in Figur 6A und 6B dargestellten Bilder ist 16 bzw. 15 *µ*m. Weitere Experimente zeigten, daß die Lipidschichten für mindestens 4 Wochen stabil sind und daß auf den Lipidschichten weitere Polyelektrolytschichten ohne Zerstörung der darunter liegenden Lipidschicht abgeschieden werden können.

Die Technik der Elektrorotation (Arnold et al., J. Phys. Chem. 91 (1987), 5093; Fuhr et al., In: Electromanipulation of Cells, U. Zimmermann und G.A. Neil, Hrsg., CRC Press, Boca Raton (1996), 259-328; Prüger et al., Biophys. J. 72 (1997), 1414) ist eine spektroskopische Methode, welche die Untersuchung der dielektrischen Eigenschaften von Mehrschichtstrukturen ermöglicht. Dabei wird ein rotierendes elektrisches Feld im KHz- bis MHz-Bereich an die Teilchensuspension angelegt. Das induzierte Dipolmoment bildet eine Winkel mit dem angelegten Feldvektor, wenn die Rotationsgeschwindigkeit des Felds zu schnell ist, daß ihm das zu induzierte Dipolmoment folgen kann. Als Ergebnis erhält das Teilchen ein Drehmoment, welches wiederum zu einer erkennbaren Drehung des Teilchens selbstführt. Ein Elektrorotationsspektrum wird durch Messung der Teilchenrotationsgeschwindigkeit als Funktion der Rotationsfrequenz des äußeren elektrischen Felds erhalten.

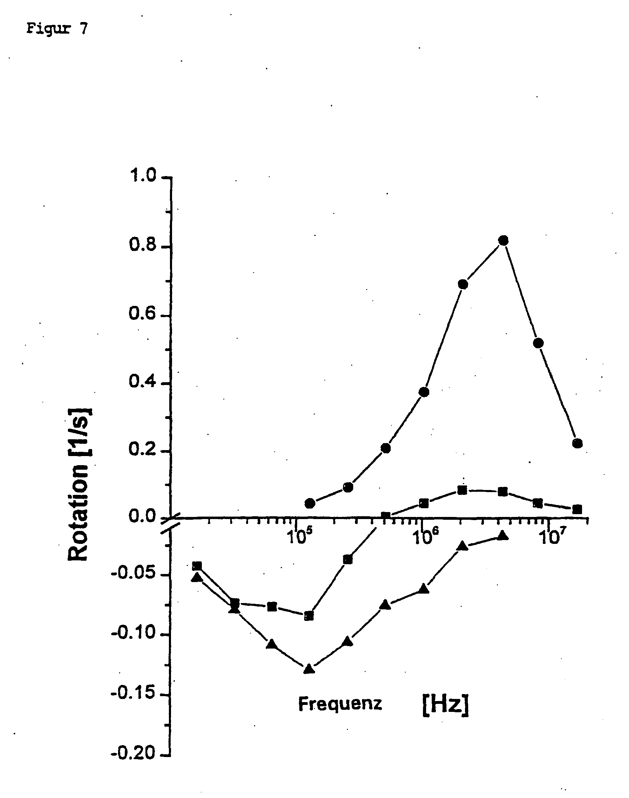

Figur 7 zeigt drei typische Elektrorotationsspektren für eine unbeschichtete Polyelektrolythülle als Kontrolle (A), eine mit DPPA beschichtete Polyelektrolythülle (B) und eine mit DPPC beschichtete Polyelektrolythülle (C). Die Leitfähigkeit der wässrigen Phase außerhalb der Hüllen war 2.000 µS/cm, 80 µS/cm bzw. 100 µS/cm. Das Vorhandensein einer isolierenden Lipidschicht führt zu einer negativen Rotationsrichtung im KHz-Bereich. Die stark leitfähige Polyelektrolythülle (etwa 10⁴ µS/cm) erzeugt den bei der Kontrolle in der MHz-Region erkennbaren positiven Peak. Die schwach leitfähige DPPA-Schicht wird bei hohen Frequenzen kurzgeschlossen. Die Abwesenheit einer positiven Rotation im Falle der DPPC-Beschichtung weist auf das Vorhandensein von Lipidmultischichten hin. Diese Ergebnisse zeigen, daß Polyelektrolyt-Multischichten mit Lipiden zur Kontrolle der Permeabilität erfolgreich beschichtet werden können und daß die beschichteten Hüllen für ionische Verbindungen wie Salze wenig durchlässig sind.

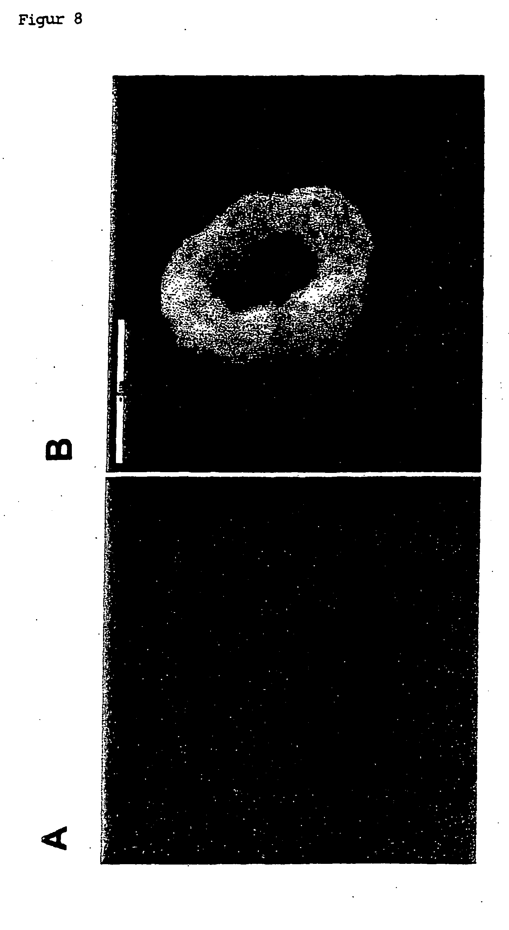

Die leeren Polyelektrolythüllen können auch zur kontrollierten Präzipitation oder Kristallisation organischer oder anorganischer Materialien verwendet werden. Hierzu werden auf Discozyten templatierte Polyelektrolythüllen in einer 30 mM 6-CF Lösung bei pH 7 inkubiert. Anschließend wurde der pH Wert rasch auf einen Wert von 3,5 verändert, bei dem 6-CF weitgehend unlöslich wird. Nach Inkubation über 1 bis 12 h wurde eine Mischung von scheinbar vollständig mit 6-CF gefüllten und leeren Hüllen erhalten. Figur 8A zeigt die lichtmikroskopische Aufnahme einer Polyelektrolythülle (10 Schichten) und Figur 8B das entsprechende rasterelektronenmikroskopische Bild. Das vollständig dunkle Bild A ist auf die starke Adsorption des kristallisierten 6-CF zurückzuführen. Das SEM-Bild zeigt, daß das im Inneren der Hülle kristallisierte 6-CF die Form des ursprünglichen Templats annimmt. Die Breite von Bild A ist 8 µm. In weiteren Experimenten wurde gezeigt, daß Rhodamin B durch Erhöhung des pH-Wertes präzipitiert werden kann. Die Präzipitation von Wirkstoffen kann auch durch andere Maßnahmen, z.B. Lösungsmittelaustausch, Salzfällung etc. ausgelöst werden. Diese Ergebnisse zeigen, daß die Polyelektrolythüllen als Template für Kristallisations- oder Präzipitationsprozesse dienen können, wodurch eine Kontrolle der Größe und Form von durch die Reaktion entstandenen kolloidalen Teilchen ermöglicht wird.

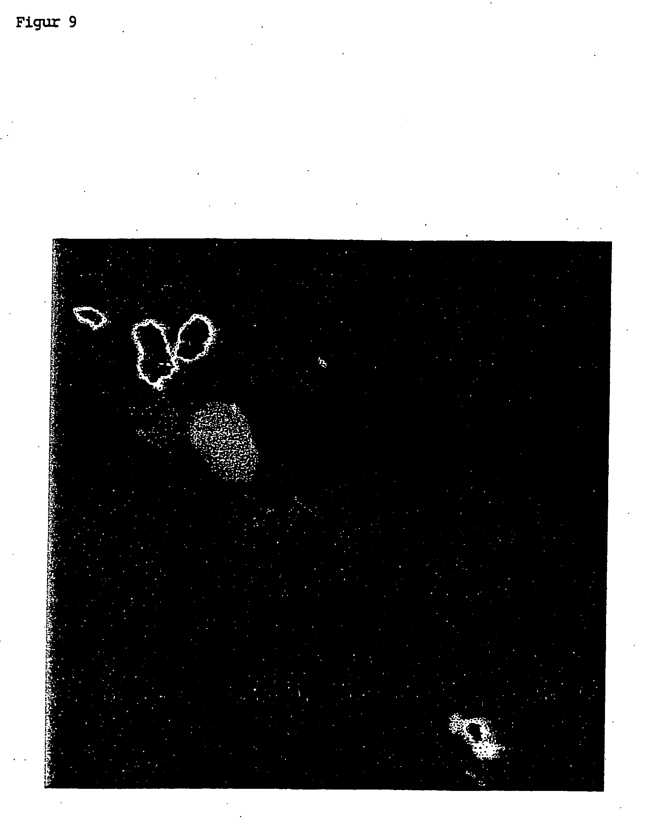

Figur 9 zeigt das Ergebnis einer radikalischen Polymerisation von Diallyldimethylammoniumchlorid (DADMAC) in PAH/PSS Hüllen. Hierzu wurde eine 3%ige Monomerlösung in einer 2%igen Kapselsuspension mit dem Polymerisationsinitiator Natriumperoxodisulfat (30 mg/100 ml) versetzt und 9,5 h bei 70°C polymerisiert. Durch Zentrifugation wurde das in der Volumenphase synthetisierte Polymer PDADMAC entfernt. Nach Behandlung mit 100 mM 6-CF ist deutlich die Bindung des Farbstoffs an die Aminogruppen von PDADMAC zu erkennen. Das Polymer ist an den negativen Kapselwänden adsorbiert, es ist aber auch im Inneren der Kapseln zu finden.

Aus neun Schichten bestehende Polyelektrolythüllen ([PSS/PAH]₄PSS) wurden auf Humanerythrozyten abgeschieden und die Templatpartikel entfernt. Anschließend wurde eine weitere Schicht PAH aufgetragen. Die Kapseln wurden zur radikalischen Polymerisation von Acrylsäure zu Polyacrylsäure verwendet. Hierzu wurde eine 3%ige Monomerlösung in einer 2%igen Kapselsuspension mit dem Initiator Natriumperoxodisulfat (30 mg/100 ml) versetzt und 9,5 h bei 70°C polymerisiert. Durch Zentrifugation wurde die in der Volumenphase synthetisierte Polyacrylsäure entfernt. Nach Zugabe von 100 nM Rhodamin B (das selektiv an anionische Gruppen bindet) konnte das Vorhandensein von Polyacrylsäure adsorbiert an die negativ geladenen Kapselwände, aber auch im Inneren der Kapseln nachgewiesen werden. Aufgrund einer durch Acrylsäure vermittelten Brückenbildung fand eine Flockung der Kapseln statt. Diese Adsorption der Acrylsäure kann durch Verwendung von Kapseln mit einer äußeren negativen Ladung verhindert werden.

## Patentansprüche

1. Verfahren zur Herstellung von Kapseln mit einer Polyelektrolythülle, die einen Durchmesser im Bereich von 10 nm bis 10 µm aufweisen, **dadurch gekennzeichnet,**
**dass** man auf einem Templat ausgewählt aus Aggregaten biologischer oder/und amphiphiler Materialien mehrere aufeinanderfolgende Schichten entgegengesetzt geladener Polyelektrolytmoleküle aufbringt, indem man zunächst eine Dispersion der Templatpartikel in einer wässrigen Lösung erzeugt, zu dieser Dispersion dann eine Polyelektrolytspezies mit der gleichen oder der entgegengesetzten Ladung wie die Oberfläche des Templatpartikels gibt, anschließend nach Abtrennung gegebenenfalls vorhandener überschüssiger Polyelektrolytmoleküle die für den Aufbau der zweiten Schicht verwendete entgegengesetzt geladene Polyelektrolytspezies zugibt und dann gegebenenfalls weitere abwechselnd entgegengesetzt geladene Schichten von Polyelektrolytmolekülen aufgebracht werden und anschließend gegebenenfalls das Templat desintegriert.

2. Verfahren nach Anspruch 1,
**dadurch gekennzeichnet,**
**dass** für jede Schicht mit gleicher Ladung gleiche oder verschiedene Polyelektrolytspezies ausgewählt werden.

3. Verfahren nach Anspruch 1 oder 2,
**dadurch gekennzeichnet,**
**dass** zwei bis vierzig Polyelektrolytschichten aufgebracht werden.

4. Verfahren nach Anspruch 3,
**dadurch gekennzeichnet,**
**dass** vier bis zwanzig Polyelektrolytschichten aufgebracht werden.

5. Verfahren nach einem der Ansprüche 1 bis 4,
**dadurch gekennzeichnet,**
**dass** man ein Templat verwendet, ausgewählt aus der Gruppe bestehend aus Zellen, Zellaggregaten, subzellulären Partikeln, Viruspartikeln und Aggregaten von Biomolekülen.

6. Verfahren nach einem der Ansprüche 1 bis 4,
**dadurch gekennzeichnet,**
**dass** man Pollen als Templat verwendet.

7. Verfahren nach einem der Ansprüche 1 bis 4,
**dadurch gekennzeichnet,**
**dass** man ein Templat verwendet, ausgewählt aus der Gruppe bestehend aus Vesikeln, Liposomen, Micellen und Lipidaggregaten.

8. Verfahren nach einem der vorhergehenden Ansprüche,
**dadurch gekennzeichnet,**
**dass** das Templat mit einem Fixierungsreagenz vorbehandelt wird.

9. Verfahren nach einem der vorhergehenden Ansprüche,
**dadurch gekennzeichnet,**
**dass** man Formaldehyd oder/und Glutardialdehyd als Fixierungsreagenz verwendet.

10. Verfahren nach einem der Ansprüche 1-9,
**dadurch gekennzeichnet,**
**dass** ein partiell desintegriertes Templat als Ausgangsmaterial zum Aufbringen der Polyelektrolytmoleküle verwendet wird.

11. Verfahren nach Anspruch 10,
**dadurch gekennzeichnet,**
**dass** das Templat eine toroidale Struktur aufweist.

12. Verfahren nach einem der vorhergehenden Ansprüche,
**dadurch gekennzeichnet,**
**dass** die Desintegration des Templats durch Zugabe eines Lysereagenz erfolgt.

13. Verfahren nach Anspruch 12,
**dadurch gekennzeichnet,**
**dass** das Lysereagenz ein Deproteinisierungsmittel, ausgewählt aus Peroxo- und Hypochloritverbindungen, enthält.

14. Verfahren nach Anspruch 13,
**dadurch gekennzeichnet,**
**dass** man Natrium- oder Kaliumhypochlorit als Deproteinisierungsmittel verwendet.

15. Verfahren nach einem der vorhergehenden Ansprüche,
**dadurch gekennzeichnet,**
**dass** nach Desintegration des Templat mindestens eine Lipidschicht auf der Polyelektrolythülle abgeschieden wird.

16. Verfahren nach Anspruch 15,
**dadurch gekennzeichnet,**
**dass** mindestens eine weitere Polyelektrolytschicht auf der Lipidschicht abgeschieden wird.

17. Verfahren nach einem der vorhergehenden Ansprüche,
**dadurch gekennzeichnet,**
**dass** ein Wirkstoff in die Kapseln eingebracht wird.

18. Verfahren nach einem der vorhergehenden Ansprüche,
**dadurch gekennzeichnet,**
**dass** der Wirkstoff aus Katalysatoren, pharmazeutischen Wirkstoffen, Polymeren, Farbstoffen, Sensormolekülen, Aromastoffen und Pflanzenschutzmitteln ausgewählt wird.

19. Verfahren nach einem der vorhergehenden Ansprüche,
**dadurch gekennzeichnet,**
**dass** organische Flüssigkeiten oder Gase in die Kapseln eingebracht werden.

20. Verfahren nach einem der vorhergehenden Ansprüche,
**dadurch gekennzeichnet,**
**dass** in oder/und auf den Kapseln eine chemische Reaktion, z.B. eine Polymersynthese, durchgeführt wird.

21. Verfahren nach einem der vorhergehenden Ansprüche,
**dadurch gekennzeichnet,**
**dass** in oder/und auf den Kapseln eine Präzipitation oder Kristallisation durchgeführt wird.

22. Polyelektrolyt-Kapseln, erhältlich durch ein Verfahren nach einem der Ansprüche 1 - 21,
**dadurch gekennzeichnet,**
**dass** sie die Templatpartikel in ihrem Inneren enthalten.

23. Kapseln nach Anspruch 22,
**dadurch gekennzeichnet,**
**dass** sie eine Wandstärke der Polyelektrolytschichten im Bereich von 2 bis 100 nm aufweisen.

24. Kapseln nach Anspruch 22 oder 23,
**dadurch gekennzeichnet,**
**dass** sie eine von den Templatpartikeln vorgegebene äußere Form aufweisen.

25. Kapseln nach Anspruch 24,
**dadurch gekennzeichnet,**
**dass** sie eine anisotrope Form aufweisen.

26. Kapseln nach Anspruch 25,
**dadurch gekennzeichnet,**
**dass** sie eine toroidale Form aufweisen.

27. Kapseln nach Anspruch 22 - 26,
**dadurch gekennzeichnet,**
**dass** sie einen Wirkstoff enthalten.

28. Verwendung von Kapseln nach einem der Ansprüche 22 - 27 zur Verkapselung von Wirkstoffen.

29. Verwendung von Kapseln nach einem der Ansprüche 22 - 27 als Mikroreaktionsräume für chemische Reaktionen oder als Präzipitations- oder Kristallisationstemplat.

30. Verwendung von Kapseln nach einem der Ansprüche 22 - 27 in der Sensorik, Oberflächenanalytik oder Informationstechnologie.

31. Verwendung von Kapseln nach einem der Ansprüche 22 - 27 in der Pharmazie und Medizin, Lebensmitteltechnologie, Kosmetik und Druckindustrie.

32. Verwendung von Kapseln nach einem der Ansprüche 22 - 27 zum Aufbau von Mikro- bzw. Nanokompositen.

33. Verwendung von Kapseln nach einem der Ansprüche 22 - 27 als Emulsionsträger.

34. Anisotrope Partikelzusammensetzungen, erhältlich durch Verkapselung von Wirkstoffen in Kapseln nach Anspruch 25 oder 26 und Entfernung der Polyelektrolythülle.

35. Zusammensetzungen nach Anspruch 34 in Form einer Dispersion.

## Claims

1. A process for producing capsules with a polyelectrolyte shell, which have a diameter in the range of from 10 nm to 10 µm, **characterised in that** several consecutive layers of oppositely charged polyelectrolyte molecules are applied to a template selected from aggregates of biological and/or amphiphilic materials by initially producing a dispersion of the template particles in an aqueous solution, then adding to this dispersion a polyelectrolyte species with the same or the opposite charge as the surface of the template particle, subsequently, after removing any excess polyelectrolyte molecules present, adding the oppositely charged polyelectrolyte species used to build up the second layer, and then where appropriate applying further alternately oppositely charged layers of polyelectrolyte molecules, and the template is subsequently disintegrated where appropriate.

2. A process according to Claim 1, **characterised in that** like or different polyelectrolyte species are selected for each layer with the same charge.

3. A process according to Claim 1 or 2, **characterised in that** two to forty polyelectrolyte layers are applied.

4. A process according to Claim 3, **characterised in that** four to twenty polyelectrolyte layers are applied.

5. A process according to any of Claims 1 to 4, **characterised in that** a template selected from the group consisting of cells, cell aggregates, subcellular particles, virus particles and aggregates of biomolecules is used.

6. A process according to any of Claims 1 to 4, **characterised in that** pollen is used as template.

7. A process according to any of Claims 1 to 4, **characterised in that** a template selected from the group consisting of vesicles, liposomes, micelles and lipid aggregates is used.

8. A process according to any of the preceding claims, **characterised in that** the template is pretreated with a fixing reagent.

9. A process according to any of the preceding claims, **characterised in that** formaldehyde and/or glutaraldehyde is used as fixing reagent.

10. A process according to any of Claims 1 to 9, **characterised in that** a partially disintegrated template is used as starting material for the application of the polyelectrolyte molecules.

11. A process according to Claim 10, **characterised in that** the template has a toroidal structure.

12. A process according to any of the preceding claims, **characterised in that** the template is disintegrated by adding a lytic reagent.

13. A process according to Claim 12, **characterised in that** the lytic reagent contains a deproteinising agent selected from peroxo and hypochlorite compounds.

14. A process according to Claim 13, **characterised in that** sodium or potassium hypochlorite is used as deproteinising agent.

15. A process according to any of the preceding claims, **characterised in that** at least one lipid layer is deposited on the polyelectrolyte shell after disintegration of the template.

16. A process according to Claim 15, **characterised in that** at least one further polyelectrolyte layer is deposited on the lipid layer.

17. A process according to any of the preceding claims, **characterised in that** an active ingredient is introduced into the capsules.

18. A process according to any of the preceding claims, **characterised in that** the active ingredient is selected from catalysts, active pharmaceutical ingredients, polymers, dyes, sensor molecules, aroma substances and crop protection agents.

19. A process according to any of the preceding claims, **characterised in that** organic liquids or gases are introduced into the capsules.

20. A process according to any of the preceding claims, **characterised in that** a chemical reaction, for example a polymer synthesis, is carried out in and/or on the capsules.

21. A process according to any of the preceding claims, **characterised in that** a precipitation or crystallization is carried out in and/or on the capsules.

22. Polyelectrolyte capsules obtainable by a process according to any of Claims 1 to 21, **characterised in that** they comprise the template particles in their interior.

23. Capsules according to any of Claim 22, **characterised in that** they have a wall thickness of the polyelectrolyte layers in the range of from 2 to 100 nm.

24. Capsules according to Claim 22 or 23, **characterised in that** they have an external shape predetermined by the template particles.

25. Capsules according to Claim 24, **characterised in that** they have an anisotropic shape.

26. Capsules according to Claim 25, **characterised in that** they have a toroidal shape.

27. Capsules according to Claims 22 to 26, **characterised in that** they comprise an active ingredient.

28. Use of capsules according to any of Claims 22 to 27 for encapsulating active ingredients.

29. Use of capsules according to any of Claims 22 to 27 as microreaction chambers for chemical reactions or as precipitation or crystallisation template.

30. Use of capsules according to any of Claims 22 to 27 in sensor systems, surface analysis or information technology.

31. Use of capsules according to any of Claims 22 to 27 in pharmacy and medicine, food technology, cosmetics and the printing industry.

32. Use of capsules according to any of Claims 22 to 27 for building up microcomposites or nanocomposites.

33. Use of capsules according to any of Claims 22 to 27 as emulsion carriers.

34. Anisotropic particle compositions obtainable by encapsulating active ingredients in capsules according to Claim 25 or 26 and removing the polyelectrolyte shell.

35. Compositions according to Claim 34 in the form of a dispersion.

## Revendications

1. Procédé de fabrication de capsules comportant une enveloppe polyélectrolyte, qui présentent un diamètre dans la plage de 10 nm à 10 µm,
**caractérisé en ce que**,
sur une matrice, choisie parmi des agrégats de matières biologiques et/ou amphiphiles, on applique plusieurs couches successives de molécules de polyélectrolyte à charge opposée, en préparant d'abord une dispersion des particules de matrice dans une solution aqueuse ; en ajoutant ensuite à cette dispersion une espèce de polyélectrolyte ayant la même charge ou une charge opposée à la surface de la particule de matrice, en ajoutant ensuite, après avoir séparé les molécules de polyélectrolyte excédentaires éventuellement présentes, l'espèce de polyélectrolyte à charge opposée utilisée pour la construction de la deuxième couche, puis en appliquant, le cas échéant, d'autres couches à charge alternativement opposée de molécules de polyélectrolyte, puis en désintégrant, le cas échéant, la matrice.

2. Procédé selon la revendication 1,
**caractérisé en ce**
**que** l'on choisit, pour chaque couche de même charge, une même espèce de polyélectrolyte ou des espèces de polyélectrolyte différentes.

3. Procédé selon la revendication 1 ou la revendication 2,
**caractérisé en ce**
**que** l'on applique deux à quarante couches de polyélectrolyte.

4. Procédé selon la revendication 3,
**caractérisé en ce**
**que** l'on applique quatre à vingt couches de polyélectrolyte.

5. Procédé selon l'une quelconque des revendications 1 à 4,
**caractérisé en ce**
**que** l'on utilise une matrice choisie dans le groupe constitué de cellules, d'agrégats de cellules, de particules subcellulaires, de particules de virus et d'agrégats de biomolécules.

6. Procédé selon l'une quelconque des revendications 1 à 4,
**caractérisé en ce**
**que** l'on utilise du pollen en tant que matrice.

7. Procédé selon l'une quelconque des revendications 1 à 4,
**caractérisé en ce**
**que** l'on utilise une matrice choisie dans le groupe constitué de vésicules, de liposomes, de micelles et d'agrégats lipidiques.

8. Procédé selon l'une quelconque des revendications précédentes,
**caractérisé en ce**
**que** l'on traite au préalable la matrice à l'aide d'un réactif de fixation.

9. Procédé selon l'une quelconque des revendications précédentes,
**caractérisé en ce que**
l'on utilise du formaldéhyde et/ou du glutaraldéhyde en tant que réactif de fixation.

10. Procédé selon l'une quelconque des revendications 1 à 9,
**caractérisé en ce**
**que** l'on utilise une matrice partiellement désintégrée en tant que matière de départ pour appliquer les molécules de polyélectrolyte.

11. Procédé selon la revendication 10,
**caractérisé en ce**
**que** la matrice présente une structure toroïdale.

12. Procédé selon l'une quelconque des revendications précédentes,
**caractérisé en ce**
**que** la désintégration de la matrice est réalisée par addition d'un réactif de lyse.

13. Procédé selon la revendication 12,
**caractérisé en ce**
**que** le réactif de lyse contient un agent de déprotéinisation choisi parmi les composés peroxo- et hypochlorite.

14. Procédé selon la revendication 13,
**caractérisé en ce**
**que** l'on utilise de l'hypochlorite de sodium ou de potassium en tant qu'agent de déprotéinisation.

15. Procédé selon l'une quelconque des revendications précédentes,
**caractérisé en ce**
**qu'**au moins une couche lipidique est déposée sur l'enveloppe polyélectrolyte après désintégration de la matrice.

16. Procédé selon la revendication 15,
**caractérisé en ce**
**qu'**au moins une autre couche de polyélectrolyte est déposée sur la couche lipidique.

17. Procédé selon l'une quelconque des revendications précédentes,
**caractérisé en ce**
**qu'**un principe actif est introduit dans les capsules.

18. Procédé selon l'une quelconque des revendications précédentes,
**caractérisé en ce**
**que** le principe actif est choisi parmi des catalyseurs, des principes actifs pharmaceutiques, des polymères, des colorants, des molécules détectrices, des substances aromatisantes et des agents phytosanitaires.

19. Procédé selon l'une quelconque des revendications précédentes,
**caractérisé en ce**
**que** l'on introduit des liquides ou des gaz organiques dans les capsules.

20. Procédé selon l'une quelconque des revendications précédentes,
**caractérisé en ce**
**qu'**une réaction chimique, par exemple une synthèse de polymères, est réalisée dans et/ou sur les capsules.

21. Procédé selon l'une quelconque des revendications précédentes,
**caractérisé en ce**
**qu'**une précipitation ou une cristallisation est réalisée dans et/ou sur les capsules.

22. Capsules en polyélectrolyte, susceptibles d'être obtenues grâce à un procédé selon l'une quelconque des revendications 1 à 21,
**caractérisées en**
**qu'**elles contiennent en leur sein les particules de matrice.

23. Capsules en polyélectrolyte selon la revendication 22,
**caractérisées en ce**
**qu'**elles présentent une épaisseur de paroi des couches de polyélectrolyte dans la plage de 2 à 100 nm.

24. Capsules en polyélectrolyte selon la revendication 22 ou 23
**caractérisées en ce**
**qu'**elles ont une forme externe prédéterminée par les particules de matrice.

25. Capsules selon la revendication 24,
**caractérisées en ce**
**qu'**elles ont une forme anisotrope.

26. Capsules selon la revendication 25,
**caractérisées en ce**
**qu'**elles ont une forme toroïdale.

27. Capsules selon l'une quelconque des revendications 22 à 26,
**caractérisées en ce**
**qu'**elles contiennent un principe actif.

28. Utilisation de capsules selon l'une quelconque des revendications 22 à 27 pour encapsuler des principes actifs.

29. Utilisation de capsules selon l'une quelconque des revendications 22 à 27 en tant qu'espaces de micro-réaction pour des réactions chimiques ou en tant que matrice de précipitation ou de cristallisation.

30. Utilisation de capsules selon l'une quelconque des revendications 22 à 27 dans le domaine des détecteurs, de l'analyse de surfaces ou de l'informatique.

31. Utilisation de capsules selon l'une quelconque des revendications 22 à 27 en pharmacie et en médecine, dans la technologie des produits alimentaires, la cosmétique et l'imprimerie.

32. Utilisation de capsules selon l'une quelconque des revendications 22 à 27 pour construire des micro-composites respectivement des nano-composites.

33. Utilisation de capsules selon l'une quelconque des revendications 22 à 27 en tant que supports d'émulsion.

34. Compositions de particules anisotropes, susceptibles d'être obtenues par encapsulage de principes actifs dans des capsules selon la revendication 25 ou 26 et enlèvement de l'enveloppe de polyélectrolyte.

35. Compositions selon la revendication 34 sous la forme d'une dispersion.
